# FASCICULE DE BREVET EUROPEEN

(11) **EP 0 662 454 B1**
(45) Date de publication et mention de la délivrance du brevet: **06.03.2002**
(21) Numéro de dépôt: 94403013.9
(22) Date de dépôt: 26.12.1994
(51) Int. Cl.: C02F 9/00, C02F 1/66, C07C 201/16

(54) **Procédé d'élimination d'effluents aqueux comprenant notamment des composés hydroxynitroaromatiques**
Verfahren zur Entfernung von hydroxynitroaromatischen Verbindungen enthaltenden Abwassers
Process to remove an aqueous effluent containing hydroxynitroaromatic compounds

(30) Priorité: 31.12.1993 FR 9315988
(43) Date de publication de la demande: 12.07.1995
(73) Titulaire: RHODIA CHIMIE, 92512 Boulogne Billancourt Cedex (FR)
(72) Inventeur: Joulak, Faouzi, F-78150 Le Chesnay (FR); Le Bris, Louis, F-69002 Lyon (FR); Marion, Philippe, F-69100 Villeurbanne (FR)
(74) Mandataire: Ricalens, François

(56) Documents cités:
- US-A- 4 597 875
- US-A- 4 604 214

## Description

La présente invention a trait à un procédé pour éliminer les effluents aqueux comprenant des composés du type hydroxynitroaromatiques.

Plus particulièrement, ce procédé trouve son application dans le procédé de préparation de composés nitroaromatiques par réaction de composés aromatiques avec l'acide nitrique en présence d'acide sulfurique.

Après la réaction de nitration des composés aromatiques, le mélange réactionnel est traité de façon à séparer les composés nitroaromatiques obtenus, des acides minéraux employés comme réactif et catalyseur, puis des sous-produits formés.

Habituellement, cette opération est constituée de plusieurs lavages successifs, suivis chacun d'un cycle décantation / séparation des phases aqueuse et organique. Ainsi, dans un premier temps, le mélange réactionnel est mis en contact avec de l'eau pour récupérer les acides nitrique et sulfurique. Puis on effectue un ou plusieurs lavages avec de l'eau additionnée d'un agent neutralisant pour éliminer les sous-produits.

Par sous-produits, on désigne notamment les composés du type hydroxyaromatiques, comprenant un, deux, ou trois groupements nitrés (comme les nitrophénols, les nitrocrésols). Ces composés représentent les substances les plus néfastes, car elles sont reconnues comme poison des catalyseurs mis en oeuvre dans la réaction ultérieure d'hydrogénation dans laquelle peuvent être engagés les composés nitroaromatiques obtenus. Cette réaction permet de réduire les groupements nitrés en groupements aminés et les produits résultants peuvent, par la suite, être utilisés comme réactifs pour obtenir des composés comme les isocyanates notamment

Le terme sous-produits peut aussi désigner les composés du type des acides carboxyliques aromatiques, comprenant un, deux ou trois groupement nitrés et des acides carboxyliques comprenant un à six atomes de carbone, comme les acides formique, acétique, oxalique.

Dans la suite de la description, les sous produits dont la liste non exhaustive vient d'être donnée, seront désignés soit par sous-produits, soit par composés hydroxynitroaromatiques.

Le problème se pose du traitement et/ou de l'élimination de la phase aqueuse récupérée après les diverses étapes de lavages correspondant à la séparation des composés nitroaromatiques des sous-produits formés, car la quantité de ces sous-produits est jugée trop importante. Par conséquent, il n'est pas possible de rejeter de tels effluents sans les avoir traités au préalable.

L'une des solutions possibles à ce problème est de collecter ces eaux et de les incinérer, éventuellement après les avoir concentrées. Cependant, ce type de méthode n'est pas viable économiquement car le coût nécessité d'une part pour concentrer les eaux et d'autre part pour les incinérer, est trop élevé. De plus, cette voie ne résout pas le problème des rejets de composés minéraux utilisés notamment pour l'étape de neutralisation.

Une autre solution envisagée est d'extraire les sous-produits, et principalement les composés hydroxynitroaromatiques, avec un solvant approprié et de concentrer puis d'incinérer les produits ainsi extraits. Cette solution a par exemple été explicitée dans le brevet américain US 4 597 875. Cependant, cette opération n'est pas non plus intéressante sur le plan économique du fait de la multiplication des étapes (extraction, concentration) et de l'emploi de composés supplémentaires.

Il est par ailleurs connu de traiter les effluents aqueux, avant leur rejet, par des voies chimiques ou biochimiques, pour détruire les sous-produits présents dans la phase aqueuse. Par exemple, dans le brevet américain US 4 604 214, il est décrit de laver le mélange comprenant lesdits composés nitroaromatiques et les composés hydroxynitroaromatiques avec une base diluée, dans le but de transformer les sous-produits en sels. Par cette opération, lesdits sous-produits deviennent hydrosolubles, au contraire des composés nitroaromatiques qui restent en phase organique, et peuvent alors être facilement séparés. Puis les eaux basiques obtenues après séparation des phases sont acidifiées et mises en contact avec le réactif de Fenton, qui est un agent oxydant, pour dégrader les composés hydroxynitroaromatiques.

Il faut noter que ces méthodes peuvent, elles aussi, être coûteuses et représentent une étape supplémentaire dans le procédé. De plus, les quantités d'eau rejetées sont très importantes, et même si elles ont été débarrassées des composés hydroxynitroaromatiques, elles contiennent néanmoins une fraction non négligeable d'agent neutralisant, perdue, qu'il est souhaitable de détruire avant le rejet des eaux.

Cependant, même si l'intérêt de tels procédés n'est pas remis en cause, il n'en reste pas moins que ce sont des procédés complexes puisqu'ils nécessitent la mise en oeuvre de plusieurs étapes, avec l'emploi de divers réactifs.

Comme on peut le constater, il n'existe à l'heure actuelle aucun procédé efficace et intéressant sur le plan économique pour résoudre ce problème des eaux usées provenant d'un procédé de nitration de composés aromatiques.

La présente invention a donc pour objet un procédé permettant d'éliminer les eaux usées issues de ce type de procédés alliant efficacité, simplicité de mise en oeuvre et coût minimisé.

En effet, la présente invention a pour objet un procédé d'élimination d'effluents aqueux contenant notamment des composés hydroxynitroaromatiques, dans lequel on met en contact un mélange de composés nitroaromatiques et notamment de composés hydroxynitroaromatiques avec un milieu lavant à base d'eau et d'un agent neutralisant, puis on sépare les phases organique et aqueuse résultantes, on met en oeuvre le procédé en continu avec un milieu lavant constitué par la phase aqueuse ainsi séparée, recyclée, et par un appoint en milieu lavant, on purge périodiquement une fraction du milieu lavant, correspondant à 3 à 10 % en poids de la quantité totale dudit milieu lavant, la quantité d'appoint en milieu lavant correspondant à la fraction purgée, on incinère ladite fraction purgée.

Ce procédé présente de nombreux avantages. En effet, il apporte la possibilité de ne rejeter aucun effluent aqueux sans, pour cela, avoir à mettre en oeuvre d'étapes nouvelles ou à utiliser de composés supplémentaires, tels que des solvants ou agents détruisant les sous-produits. De plus, le fait de recycler les eaux de lavages du milieu réactionnel est un moyen diminuant considérablement les pertes en agent neutralisant et rendant l'incinération ultérieure des effluents tout à fait attractive économiquement.

Par ailleurs, cette méthode améliore l'efficacité de la séparation des sous-produits des composés nitroaromatiques en ce sens que les pertes desdits composés nitroaromatiques par entraînement dans la phase aqueuse sont diminuées et que la concentration en sous-produits entraînés dans la phase organique avec la phase aqueuse, est abaissée.

En outre, le procédé selon l'invention peut être mis en oeuvre en discontinu et en continu. Dans ce dernier cas, il peut être intégré au procédé global d'une façon très simple, sans que ce traitement modifie la capacité des composés nitroaromatiques à être hydrogénés.

Mais d'autres avantages et caractéristiques de l'invention apparaîtront plus clairement à la lecture de la description et des exemples concrets qui vont suivre.

Ainsi, le procédé selon l'invention consiste à recycler le milieu lavant utilisé pour la séparation des composés nitroaromatiques des sous-produits, formés au cours de la réaction de nitration de composés aromatiques par l'acide nitrique en présence d'acide sulfurique.

Le mélange issu de la réaction de nitration précitée, traité selon l'invention, a été au préalable lavé à l'eau de façon à extraire les acides minéraux utilisés pour la réaction.

La phase organique résultante, constituée par le mélange des composés nitroaromatiques et hydroxynitroaromatiques - sous-produits - (dénommé par la suite, mélange à traiter), est ensuite mise en contact avec un milieu lavant à base d'eau et d'un agent neutralisant.

D'une façon classique, l'agent neutralisant est choisi parmi les composés susceptibles de transformer en sels hydrosolubles les sous-produits.

A titre d'exemple d'agent neutralisant convenant à la mise en oeuvre du procédé selon l'invention, on peut citer les hydroxydes, les carbonates, les bicarbonates de métaux alcalins ou alcalino-terreux, l'ammoniaque ; ces composés étant utilisés seuls ou en mélange.

De préférence, on utilise les hydroxydes de métal alcalin ou l'ammoniaque.

L'agent neutralisant peut se présenter sous forme pure ou diluée dans tout solvant approprié, l'eau étant préférée.

Dans le cas d'une utilisation de l'agent neutralisant sous une forme diluée, la concentration dudit agent peut varier dans une large gamme. Elle est habituellement comprise entre 0,1 et 50 % exprimé en poids et plus particulièrement comprise entre 2 et 20 %.

La quantité d'agent neutralisant mise en jeu dans le procédé est de préférence telle que le pH de la phase aqueuse soit maintenu dans une zone où les sous-produits se trouvent sous une forme de sels.

De préférence, le pH de la phase aqueuse est compris entre 6 et 8.

Il est à noter qu'opérer dans des conditions pour lesquelles le pH de la phase aqueuse serait supérieur à la valeur indiquée est envisageable, bien que cela n'apporte pas d'avantage particulier.

Dans une première variante du procédé selon l'invention, le mélange à traiter, peut comprendre en outre un co-solvant des composés nitroaromatiques fabriqués.

Plus particulièrement, on peut citer les composés aromatiques en C₆-C₁₀, éventuellement substitués par des radicaux hydrocarbonés, et/ou halogénés. A titre d'exemple, on peut mentionner le toluène, le cumène, le chlorobenzène.

Dans une seconde variante du procédé selon l'invention, le mélange à traiter ne comprend pas de co-solvant.

L'opération peut être réalisée en une ou plusieurs étapes, sous agitation.

D'une façon classique, la mise en contact du mélange et du milieu lavant est effectuée à une température variant entre 20 et 90 °C. Dans le cas plus particulier où le mélange ne comprend pas de co-solvant, la température est, de préférence, comprise entre 60 et 80°C.

Cette opération est effectuée à une pression comprise entre 0,5 et 10 bar. D'une façon avantageuse, celle-ci est voisine de la pression atmosphérique.

Le temps de contact durant chaque étape est de l'ordre de quelques minutes à 2 heures.

Après la mise en contact du mélange à traiter et du milieu lavant, on procède à une décantation du mélange résultant et l'on sépare la phase organique, comprenant les composés nitroaromatiques purifiés, de la phase aqueuse à base des sous-produits sous forme de sels.

Un ou plusieurs lavages peut avoir lieu selon le procédé de l'invention.

La caractéristique essentielle de l'invention réside dans le fait que le milieu lavant est constitué par la phase aqueuse récupérée, et recyclée dans le processus de lavage. Par ailleurs, une fraction dudit milieu lavant est purgée périodiquement avant d'être incinérée.

Selon un mode particulier de réalisation de l'invention, le milieu lavant est recyclé de telle sorte que la concentration en sous-produits soit augmentée de 10 fois par rapport à la quantité présente dans la phase aqueuse à l'issue du premier lavage. De préférence, le recyclage est effectué de telle sorte que la concentration en sous-produits soit augmentée d'au moins 20 fois et jusqu'à 30 fois.

Il est à noter qu'opérer dans des conditions telles que la concentration se trouve en dehors des valeurs indiquées serait possible. Cependant, si la concentration est augmentée de moins de 10 fois, les avantages dus au recyclage sont moins importants. Par ailleurs, si la concentration est supérieure à 30 fois la concentration initiale, on pourrait s'attendre à un risque de pollution de la phase organique par transfert des sous-produits dans cette phase organique, causant par la suite des difficultés pour la réaction d'hydrogénation.

La fraction purgée représente de 3 à 10 % en poids de la quantité totale du milieu lavant.

Le procédé selon l'invention étant de préférence mis en oeuvre en continu, la purge d'une fraction du milieu lavant nécessite un appoint de milieu lavant, correspondant à la quantité purgée. Il est à noter que ceci constitue un avantage supplémentaire du procédé. En effet, contrairement aux procédés classiques ne comprenant pas de recyclage, la quantité de milieu lavant introduite dans le procédé est considérablement plus faible que celle utilisée habituellement (3 à 10 %).

Cet appoint peut être réalisé en tout endroit de la zone du procédé dans laquelle on effectue le lavage du mélange des composés nitroaromatiques et des sous-produits.

L'appareillage dans lequel l'opération de lavage est mise en oeuvre est tout à fait classique et l'homme du métier peut notamment prévoir l'utilisation d'un ou plusieurs réservoirs de stockage pour le milieu lavant, se situant entre deux bacs de lavage.

La phase organique séparée de la phase aqueuse, après la ou les étapes de lavage, peut, si nécessaire, être lavée avec de l'eau, de façon à retirer toute trace d'agent neutralisant, notamment

L'eau résultante, comprenant éventuellement des traces d'agent neutralisant, peut être rejetée après avoir subi, le cas échéant, un traitement de purification.

Cependant, d'une façon avantageuse, cette eau de lavage est recyclée dans le procédé de l'invention. Plus particulièrement, le flux peut être réintroduit à la première étape de lavage consistant à éliminer les acides minéraux des produits issus de la réaction de nitration.

Habituellement, le procédé selon l'invention est réalisé sous agitation, à une température comprise entre 20 et 90 °C, pour une durée variant de quelques minutes à 2 heures.

Le mélange résultant est ensuite décanté puis la phase organique comprenant les composés nitroaromatiques, est séparée de la phase aqueuse.

Lesdits composés nitroaromatiques purifiés peuvent ensuite être utilisés pour la préparation de diamines aromatiques.

Les composés nitroaromatiques, après avoir éventuellement subi une étape de séchage par tout moyen connu, sont mis à réagir avec de l'hydrogène, pur ou dilué, en présence d'un catalyseur classique d'hydrogénation, comme par exemple le nickel de Raney ou encore des catalyseurs à base de platine.

De façon générale, la température de réaction se situe entre 80 et 200 °C et la pression durant la réaction est comprise entre 1 et 150 bar.

Un exemple concret mais non limitatif de l'invention va maintenant être présenté.

### EXEMPLE

Dans un réacteur agité, continu, suivi d'un décanteur, on introduit en parallèle un mélange de dinitrotoluènes contenant des trinitrocrésols et une solution diluée d'ammoniaque.
La température est de 70 °C et le temps de séjour dans la zone de lavage et de décantation est d'une heure.

Les dinitrotoluènes ont au préalable subi une première étape de lavage dans le but d'éliminer les acides minéraux présents lors de la réaction de nitration.

Dans ce mélange, la quantité d'espèces organiques acides, comprenant pour la majeure partie, des nitrocrésols, est de 1300 ppm.

Les débits en dinitrotoluènes et en ammoniaque diluée sont respectivement de 500 g/h et de 150 g/h.

La phase aqueuse en sortie du décanteur est recyclée au minimum à 90 % et elle est réintroduite dans le réacteur avec un débit de recyclage de 135 g/h.
Une fois le recyclage commencé, le débit d'ammoniaque est diminué pour correspondre à 10 % du débit total, soit 15 g/h. Cet appoint est réalisé de façon à maintenir constant le pH de la phase aqueuse dans une gamme de 7-8.

Une purge correspondant à l'apport en agent neutralisant est effectuée en continu pour être incinérée.

Avec ce mode opératoire, on augmente la concentration en sous-produits (nitrocrésols, nitrophénols, acides nitrobenzoïques) de même que celles en produits minéraux (ions ammonium provenant de l'agent neutralisant, nitrates, sulfates résiduaires non extraits lors de la première étape de lavage).
La concentration en sous-produits atteinte dans ce cas est de 1%.

Les nitrotoluènes ainsi obtenus sont lavés à 70 °C par de l'eau dans un second réacteur suivi d'une décanteur avant d'être envoyé dans un nouveau réacteur pour effectuer l'hydrogénation.

Un test d'hydrogénabilité des nitrotoluènes résultants a été réalisé afin d'en contrôler la qualité.
Ce test consiste à introduire les nitrotoluènes dans un réacteur agité comprenant de l'isopropanol, du nickel de Raney, à une température de 130 °C sous hydrogène avec une pression totale de 35 bar.
La vitesse d'hydrogénation est obtenue par mesure de la pression d'hydrogène qu'il faut introduire dans le réacteur de façon à maintenir la pression totale de 35 bar.
A l'issue de ce test, on constate que la vitesse d'hydrogénation des nitrotoluènes obtenus selon le procédé de l'invention est identique à celle de nitrotoluènes obtenus en effectuant les mêmes étapes de lavage sans qu'il y ait de recyclage.
Par conséquent, le procédé selon l'invention n'affecte pas la qualité des nitrotoluènes obtenus.

## Revendications

1. Procédé d'élimination d'effluents aqueux contenant notamment des composés hydroxynitroaromatiques, dans lequel on met en contact un mélange de composés nitroaromatiques et notamment de composés hydroxynitroaromatiques avec un milieu lavant à base d'eau et d'un agent neutralisant, puis on sépare les phases organique et aqueuse résultantes, on met en oeuvre le procédé en continu avec un milieu lavant constitué par la phase aqueuse ainsi séparée, recyclée, et par un appoint en milieu lavant, on purge périodiquement une fraction du milieu lavant, correspondant à 3 à 10 % en poids de la quantité totale dudit milieu lavant, la quantité d'appoint en milieu lavant correspondant à la fraction purgée, on incinère ladite fraction purgée.

2. Procédé selon la revendication précédente, **caractérisé en ce que** l'on utilise en tant qu'agent neutralisant les hydroxydes, carbonates, bicarbonates de métaux alcalins et/ou alcalino-terreux ainsi que l'ammoniaque.

3. Procédé selon la revendication précédente, **caractérisé en ce que** la teneur en agent neutralisant, utilisé sous forme diluée, est comprise entre 0,1 et 50 % en poids et de préférence, entre 2 et 20 % en poids.

4. Procédé selon l'une des revendications précédentes, **caractérisé en ce que** l'on maintient un pH lors de la mise en contact du mélange et du milieu lavant compris entre 5 et 8 et de préférence entre 6 et 8.

5. Procédé selon l'une des revendications précédentes, **caractérisé en ce que** l'on effectue la mise en contact à une température variant entre 20 et 90°C et de préférence entre 60 et 80 °C.

6. Procédé selon l'une des revendications précédentes, **caractérisé en ce que** le mélange à traiter comprend un co-solvant des composés nitroaromatiques, comme les composés aromatiques en C₆-C₁₀, éventuellement substitués par des radicaux hydrocarbonés, et/ou halogénés.

7. Procédé selon l'une des revendications 1 à 5, **caractérisé en ce que** le mélange à traiter ne comprend pas de co-solvant.

## Patentansprüche

1. Verfahren zur Beseitigung von insbesondere hydroxynitroaromatische Verbindungen enthaltendem Abwasser, in welchem man eine Mischung nitroaromatischer Verbindungen und insbesondere hydroxynitroaromatischer Verbindungen mit einem Waschmedium auf der Grundlage von Wasser und einem Neutralisationsmittel in Kontakt bringt, man dann die resultierenden organischen und wässrigen Phasen trennt, man das Verfahren kontinuierlich mit einem Waschmedium, das aus der so abgetrennten wässrigen Phase, welche rückgeführt wird, und einer zusätzlichen Menge des Waschmediums besteht, ablaufen lässt, man periodisch eine Fraktion des Waschmediums spült, welche 3 bis 10 Gew.-% der Gesamtmenge des genannten Waschmediums entspricht, wobei die zusätzliche Menge des Waschmediums der gespülten Fraktion entspricht, und man die genannte gespülte Fraktion einäschert.

2. Verfahren nach dem vorhergehenden Anspruch, **dadurch gekennzeichnet, dass** man als Neutralisationsmittel die Hydroxide, Carbonate, Bicarbonate von Alkalimetallen und/oder Erdalkalimetalle sowie Ammoniak verwendet.

3. Verfahren nach dem vorhergehenden Anspruch, **dadurch gekennzeichnet, dass** der Gehalt an Neutralisationsmittel, welches in verdünnter Form verwendet wird, zwischen 0,1 und 50 Gew.-% und vorzugsweise zwischen 2 und 20 Gew.-% liegt.

4. Verfahren nach einem der vorhergehenden Ansprüche, **dadurch gekennzeichnet, dass** man zur Zeit des Inkontaktbringens der Mischung und des Waschmediums einen pH aufrechterhält, welcher zwischen 5 und 8 und vorzugsweise zwischen 6 und 8 liegt.

5. Verfahren nach einem der vorhergehenden Ansprüche, **dadurch gekennzeichnet, dass** man das Inkontaktbringen bei einer Temperatur bewirkt, die zwischen 20 und 90°C und vorzugsweise zwischen 60 und 80°C variiert.

6. Verfahren nach einem der vorhergehenden Ansprüche, **dadurch gekennzeichnet, dass** die zu behandelnde Mischung ein Cosolvens der nitroaromatischen Verbindungen wie aromatische Verbindungen mit C₆-C₁₀, die eventuell durch Kohlenwasserstoffgruppen substituiert und/oder halogeniert sind, umfasst.

7. Verfahren nach einem der Ansprüche 1 bis 5, **dadurch gekennzeichnet, dass** die zu behandelnde Mischung kein Cosolvens umfasst.

## Claims

1. A process for eliminating aqueous effluents containing mainly hydroxynitroaromatic compounds, in which a mixture of mitroaromatic compounds and mainly hydroxynitroaromatic compounds is brought into contact with a water-based washing medium and a neutralising agent, then the resulting organic and aqueous phases are separated, the process being carried out continuously with a washing medium constituted by the separated aqueous phase, which is recycled, and by an addition of fresh washing medium, a fraction of washing medium corresponding to 3% to 10% by weight of the total quantity of said washing medium is purged periodically, the quantity of added washing medium corresponding to the purged fraction, and said purged fraction is incinerated.

2. A process according to the preceding claim, **characterised in that** the neutralising agent is an alkali and/or alkaline-earth metal hydroxide, carbonate or bicarbonate, or ammonia.

3. A process according to the preceding claim, **characterised in that** the quantity of neutralising agent, used in the diluted form, is in the range of 0.1 % to 50 % by weight, preferably in the range of 2 % to 20 % by weight.

4. A process according to any one of the preceding claims, **characterised in that** the pH during contact of the mixture and washing medium is in the range of 5 to 8, preferably in the range of 6 to 8.

5. A process according to any one of the preceding claims, **characterised in that** contact is carried out at a temperature of between 20°C and 90°C, preferably between 60°C and 80°C.

6. A process according to any one of the preceding claims, **characterized in that** the mixture to be treated comprises a co-solvent for nitroaromatic compounds, such as C₆-C₁₀ aromatic compounds, optionally substituted by hydrocarbon and/or halogen radicals.

7. A process according to any one of claims 1 to 5, **characterized in that** the mixture to be treated contains no co-solvent.
